Europäisches Patentamt

European Patent Office

Office européen des brevets

(11) Publication number: **0 447 708 A2**

# EUROPEAN PATENT APPLICATION

(21) Application number: 90303902.2

(51) Int. Cl.5: **A61B 5/00, G01N 21/17**

(22) Date of filing: **11.04.90**

(30) Priority: **17.03.90 CN 90101352**

(43) Date of publication of application:
**25.09.91 Bulletin 91/39**

(84) Designated Contracting States:
**DE FR GB**

(71) Applicant: **LONG XING MESD CO. HAI DIEN BEIJING**
**No. 16 Dajing Hutong, Hai Dian District**
**Beijing(CN)**

(72) Inventor: **Liu, Changxing**
**No. 16, Dajing Hutong**
**Hai Dian District, Beijing(CN)**
Inventor: **Ma, Yue**
**No. 16, Dajing Hutong**
**Hai Dian District, Beijing(CN)**

(74) Representative: **LLOYD, Patrick Alexander Desmond et al**
**Reddie & Grose 16 Theobalds Road**
**London WC1X 8PL(GB)**

(54) Apparatus and method for quantitative examination and high-resolution imaging of human tissue.

(57) A device and method for quantitative examining and for high-resolution imaging of human tissue, especially the breast, utilizing non-ionizing radiation, diffused light source with a wide spectral bandwidth in the visible and near infrared, which generates transillumination light passing through the tissue. The diffused light source is used in conjunction with a scaled compression plate for moving examination. The transilluminated light passing through the tissue is received by a CCD detector having light filters and being placed at certain angles to the light source, and is converted and processed into images by computer, which outputs not only images but also the density difference between the normal and lesion areas, projected area and volume of the lesion, and provides physicians with quantitative information for diagnosis. The intensity of the light source is flexibly controlled while the images are being fixed and stored whenever necessary. The device and method result in a convenient, rapid and highly accurate examination of human tissue and reduce discomfort during diagnosis.

FIG. 1

EP 0 447 708 A2

The present invention relates to a device and method for medical diagnosis, especially to a device and method for examining and for high-resolution imaging of human tissue, utilizing non-ionizing radiation with a wide spectral bandwidth. A diffused light source emits visible and near infrared light to transilluminate human tissue; a CCD detector is employed to receive the light signals passing through the tissue and input the electrical signals which have been converted by the CCD into a computer processing system; then a quantitative measurement for diagnosing the diseased tissue is outputted by means of the image processing system.

U.S. Pat. No. 4,649,275 disclosed a high-resolution breast imaging device using non-ionizing radiation of narrow spectral bandwidth. A collimated light source of narrow bandwidth is arranged to produce a small beam or a number of beams of very small spatial dimensions, which are performed together with two compression plates in order to obtain images of the breast. An optical detector corresponding to each source is located perpendicular to the breast to receive the collimated light passing through the breast and to convert the optical signals into electrical signals for processing. Since two transparent plates are used to compress the breast in between, this approach may entail discomfort to the examinee and inconvenience of operation, and also it is unable to examine the axillary area of the breast where lesions are of most common ocurrence. Further, as the stationary device will not permit an examination in motion, lesions in movable status can hardly be detected, and lesions in the overlapping state may be misdiagnosed. Particularly, such a collimated light source with narrow bandwidth in the form of a beam having small spatial dimension cannot illuminate a large examination area, so that a spatial image in a large scale can not be obtained at the same time, nor can a comparative analysis between the normal add the lesion regions be processed. With this method, the detector will be easily saturated and will produce poor grey scale and less contrast images, thus the advanced abilities of the computer in image processing, analysis and diagnosis will be limited.

Another prior art technique in the field uses a collimated light source with a wide spectral bandwidth in the form of a broad beam to illuminate the breast, and disposes a video camera to record the transmitted light passing through the breast and then to display, process and analyze the signals. With this method, an optical detector is placed on the opposite side of the examined breast perpendicular to the light source. The collimated light through the breast will be directly received by the optical detector and easily cause the saturation of the detector, resulting in a poorly configured grey scale image. Because the absorption of the broad beam with such a wide spectral bandwidth varies between different types of tissue, this method shows poor ability to discriminate various tissue-types. Moreover, the light scattered on the surface of the breast and detected by the detector will reduce the spatial resolution of images . As a result, the lesion sizes that are detectable with such a method have generally been no smaller than what the physician can detect by palpation.

The invention is defined in the claims to which reference should now be made.

After many experiments, studies and analyses, the present invention was created, which utilizes non-ionizing radiation with a wide spectral bandwidth in the visible and infrared to generate a diffused light with a diffuser and to enable a large area to be transilluminated. The diffused light is absorbed, refracted, reflected and scattered by the examined tissue, and results in even radiation in the illuminated area. A CCD detector is placed by the side of the tissue at certain angles to the normal of the diffuser surface. The transmitted light through the tissue is received by the CCD detector and processed into images for examination. With this approach, the images obtained are of fine definition, clear shade of grey and high resolution, and the CCD detector is not so easily saturated, while the advantages of image processing, analysis and comparative calculation are fully utilized by the computer so as to provide the physician with density differences between the normal and lesion regions for quantitative diagnosis.

The purpose of the present invention is to overcome shortcoming of prior art techniques, and to provide a device and quantitative method for examining human tissue and for high-resolution imaging. The method according to the present invention involves using a diffused light with the wide spectral bandwidth to transilluminate human tissue; a CCD detector which is placed at certain angles to the light source for obtaining images of the examined region with the quality of distinctive contrast and fine shade of grey; and a computer system which is employed to digitalize the images and to output the density difference between the normal and diseased regions from the same image for the diagnosis. The apparatus according to the present invention comprises a light source probe which is small in size and light in weight, a CCD detector with selected filters, as well as a computer system. The light source probe emitting diffused light is easy to hold and flexible to operate, and is used in conjunction with a scaled compression plate to clearly transilluminate the examined tissue. The compression plate with a handle has a measure scale in the central part which will be read together with the examined region by the CCD detector. The computer system enables accurate calculations of actual dimension, projected area and volume of the lesion according to the measurement of the scaled plate. The CCD detector receives images of the transilluminated tissue through selected filters, converts and inputs the signals into the computer system to be processed and analyzed, wherein the suspect area will be compared with the normal area. Finally the computer system outputs the density difference.

Specifically, the present invention adopts non-ionizing radiation with a wide spectral bandwidth which generates visible and near infrared light, and which can transilluminate a fairly large area through an optical diffuser, for example the entire breast. In order to have an even path of transillumination, a scaled transparent plate is used to compress the breast. Now the breast is evenly transilluminated by the diffused light with the wide spectral bandwidth, and the CCD detector is disposed to receive the light passing through the breast. The axis of the CCD detector is situated at certain angles to the normal of the diffuser surface, generally at an angle of 50 to 110 degrees, but 90 degrees is preferred. The tissue surface which the scaled plate is pressing on is positioned essentially perpendicular to the axis of the CCD detector. This approach can be compared to what people observe when the dust in the air is illuminated: standing aside from the sun light the status of the dust can be identified more clearly. Concerning the requirements for different cases, the light probe can be moved around and the breast can be illuminated from various directions, so as to detect distinctly the overlapping lesions as well as the movable characteristics of diseased areas. In order to obtain clear images of each type of breast -- breast types vary in containing more or less fat and having big or small mammary glands -- a few cut-off filters with selected wavelengths are disposed in front of the CCD detector. These filters have the cut-off wavelength of 550nm, 650nm, and 700nm respectively. The light signals of the detected region are received by the CCD detector with the filters, and are converted into electrical signals to be inputted into computer for processing. When the examined breast, has a small mammary gland and less fat, a filter with the transmitting wavelength greater than 700 nm is selected; when the breast under examination has a big mammary gland and more fat, a filter with the transmitting wavelength greater than 550nm is selected. Since various types of tissues such as fat, gland and tumor differ significantly in absorption characteristics with respect to infrared light, permitting the visible red and yellow light to be elicited by the CCD detector can increase the light flux, and can therefore raise the singal-noise ratio for imaging. The computer system processes the elicited images and displays them precisely on the screen. For providing the physician with quantitative information to diagnose the diseased tissue, the image is digitalized, the density of the normal region and the density of the diseased region are compared, and finally the density difference between the two regions within the same image is outputted.

In accordance with the present invention, the light soure probe comprises a light bulb, a reflecting cup, an optical diffuser, a light shield, a cooling fan, and a sealed heat shield. The light probe is small in size and light in weight, and is advantageous to be held and to be operated in conjunction with the scaled compression plate. The heat and the far infrared light generated by the probe have been successfully reduced to prevent examinees from being hurt by high temperature and by far infrared light. The reflecting cup is situated behind the light bulb like a parabolic mirror, which enables the light emitted to be reflected straight forward as a collimated light. The cup is coated with reflecting film and is manufactured with cooling holes. The holes on the reflecting cup will allow the cooling air to flow through and eventually to be discharged by the cooling fan sitting behind the cup. With this design, the efficiency of cooling is enhanced, and the size and the weight of the light probe can be reduced; otherwise, the dimension of the probe has to be big enough to leave a certain space between the reflecting cup and the light shield for air flowing through. The light shield at the front part of the probe is also manufactured with cooling grooves which perform the same function as the holes on the reflecting cup to allow cooling air to flow through.

In accordance with the present invention, the light probe is equipped with image fixation and light intensity control facilities which are connected with the computer processing system. During the examination, images observed can be fixed and stored with the probe at any time needed. The light intensity of the probe is adjustable up to 8 levels, so different types of breasts can be examined with different levels of light intensity. With this approach, not only the time used for searching the needed images can be saved, but also high-resolution images can be obtained with whatever types of breasts.

According to the present invention, operations of the computer system are selected with a menu-driven mode. The examiner can select the function he needs rapidly. An audio-instruction system is employed to speed up the function selection and to avoid misoperation, and can be used to train new examiners.

By use of a diffused light source, the present invention is advantageous because the CCD detector is not easy to be saturated, a large area of tissue can be transilluminated, a good signal-noise ratio for imaging can be achieved by receiving signals through the selected filters, and therefore, images of high resolution in fine shade of grey and with distinct contrast can be obtained. This is advantageous to computer processing and analysis, so that eventually the computer system will output a density difference between the normal and the diseased areas from the same image. This will provide the physician with quantitative information and help him to diagnose the lesion and to enhance the accuracy of tissue examination. Besides, owing to the small size, light weight and cooling effectiveness of the light probe, it can be easily held and operated; the examined area can be flexibly illuminated from various directions during the performance of a moving examination; the axillary region of the breast, which can be detected only with difficulty by other similar methods and devices, can be conveniently examined; the movable status of lesions can be observed; the nature of tumors can be discriminated; and missing and misdiagnosis of the overlapping lesions can be avoided. In particular, very small diseased areas which are impossible to detect by palpation become detectable with the present method and apparatus, realizing an early diagnosis of the breast lesions. Again, as the light intensity and image fixation are controlled freely during the examination, the examiner can rapidly grasp and store the desired image, and the examination can result in a shorter time for the performance, a higher resolution for imaging, and more accuracy of diagnosis. With the method and device of the present invention, the examined breast only needs to be pressed slightly, and does not need to be tightly compressed between two plates as by other existent techniques, and this can reduce discomfort to the examinee, while the whole examination procedure becomes simple, convenient, and easily accepted by examinees.

The advantages and features of the present invention will become more apparent by reference to the following description of the invention in conjunction with the accompanying drawings, wherein:

Fig. 1 is a functional block diagram of the apparatus of the present invention;
Fig. 2 contains two schematic drawings of the light source probe of the present invention;
Fig. 3 contains two schematic drawings of the reflecting cup in the light probe, 3A is the exploded view and 3B the forming graph;
Fig. 4 contains two schematic drawings of the light shield in the light probe;
Fig. 5 is a schematic drawing of the scaled compression plate;

Fig. 6 contains a group of curves of transmitting light wavelengths respective to each cut-off filter;
Fig. 7 is a spectral response region of the CCD detector array;
Fig. 8 is a schematic drawing of the placing method and positions of the light source and CCD detector;
Fig. 9 is a density difference distribution chart of tissue lesions which were calculated by the computer system and created by the present invention;
Fig. 10 is a stream diagram of the menu-driven system in support of the system operation.

The same numbers used in different figures, represent the same content.

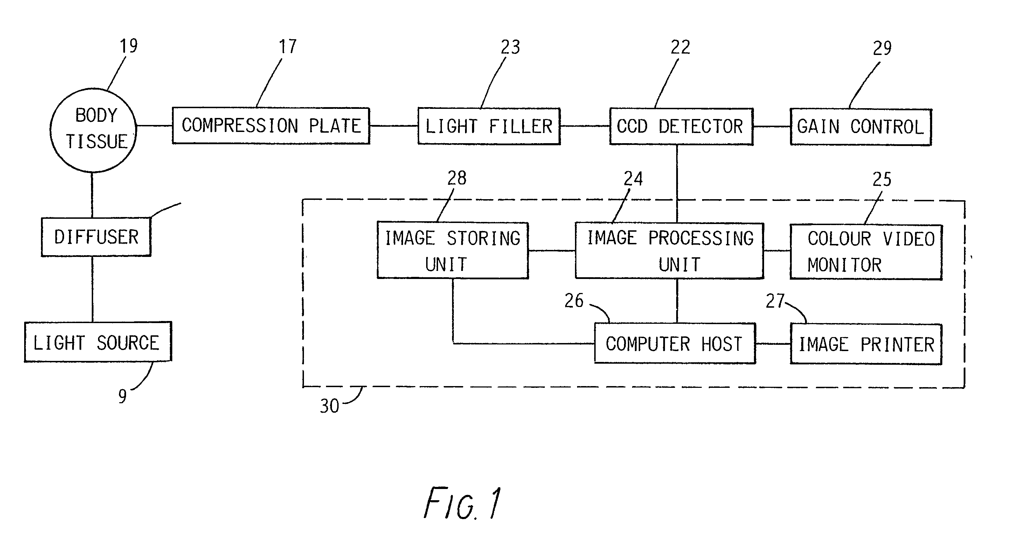

Fig. 1 is the functional block diagram of the present invention. The light source 9 is of non-ionizing radiation with a wide spectral bandwidth. It emits diffused light generating through a diffuser 1 to transilluminate human tissue 19. The compression plate 17 will be slightly pressed on the area being examined, which will enable the CCD detector 22 with a selected filter 23, placing in between, to obtain a high resolution image. Normally, the CCD detector is situated about one meter from the examined area. 30 is a computer processing system. Signals caught by CCD detector 22 are handled by three image processing boards 24, and then input into the computer 26. After being processed by the computer 26, the signals are inputted into the image storage unit 28, further into the image printer 27. After handling by the image processing boards 24, images are displayed on the colour monitor 25 by means of digital-analogue converter. 29 is a gain controller of CCD detector.

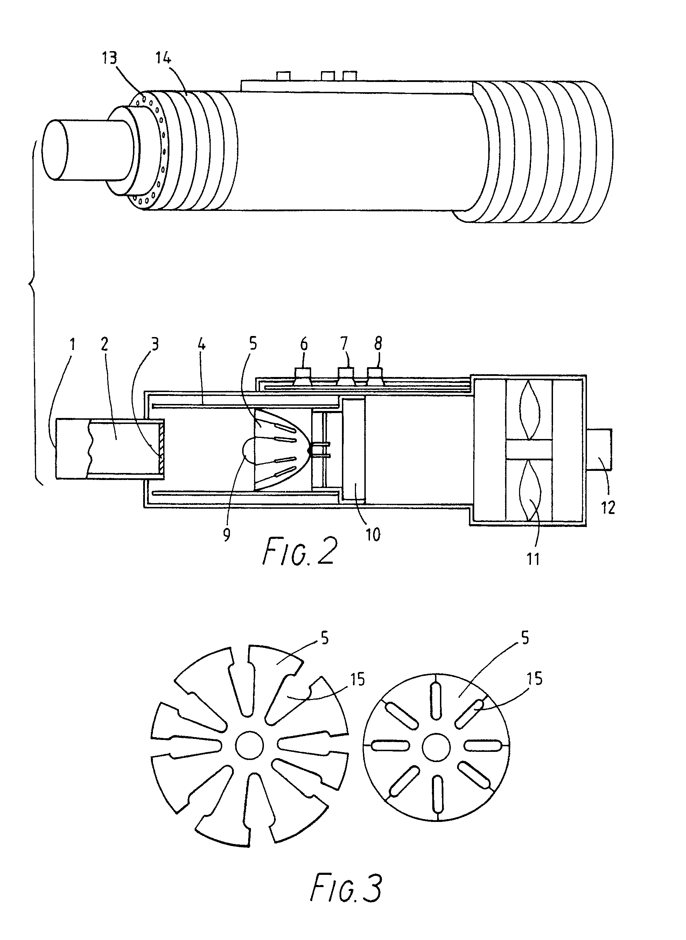

Fig. 2 is the schematic drawings of the light probe. The diffuser 1 is the exit of the diffused outgoing light; 2 is a sealed heat shield; 3 is a piece of optical glass which prevents heat conduction; 4 is a light shield; 5 is a reflecting cup; 6 is an image fixation control button; 7 and 8 are the light intensity control buttons, which respectively increase and decrease light intensity; 9 is a light bulb; 10 is a holder; 11 is an axis-flow cooling fan; 12 is a cable connector; 13 are cooling holes, and 14 is an exit of heat.

Fig. 3 shows schematic drawings of the reflecting cup. The total area of the cooling holes 15 occupies about 15% to 30% of the general area of the cup; 25% of the area is preferable.

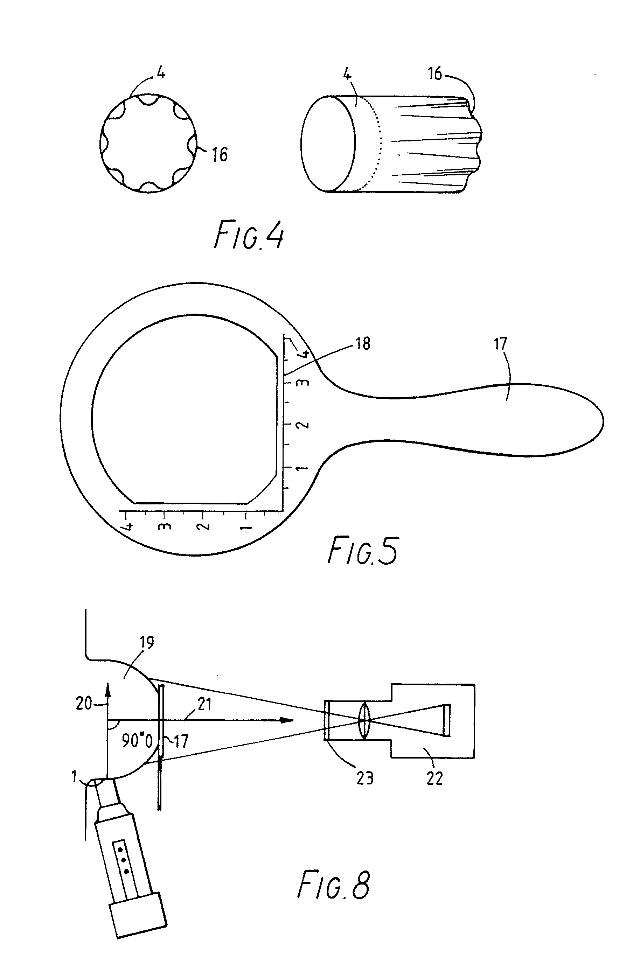

Fig. 4 shows schematic drawings of the light shield. There are cooling grooves 16 on the light shield, which is painted with a dark colour.

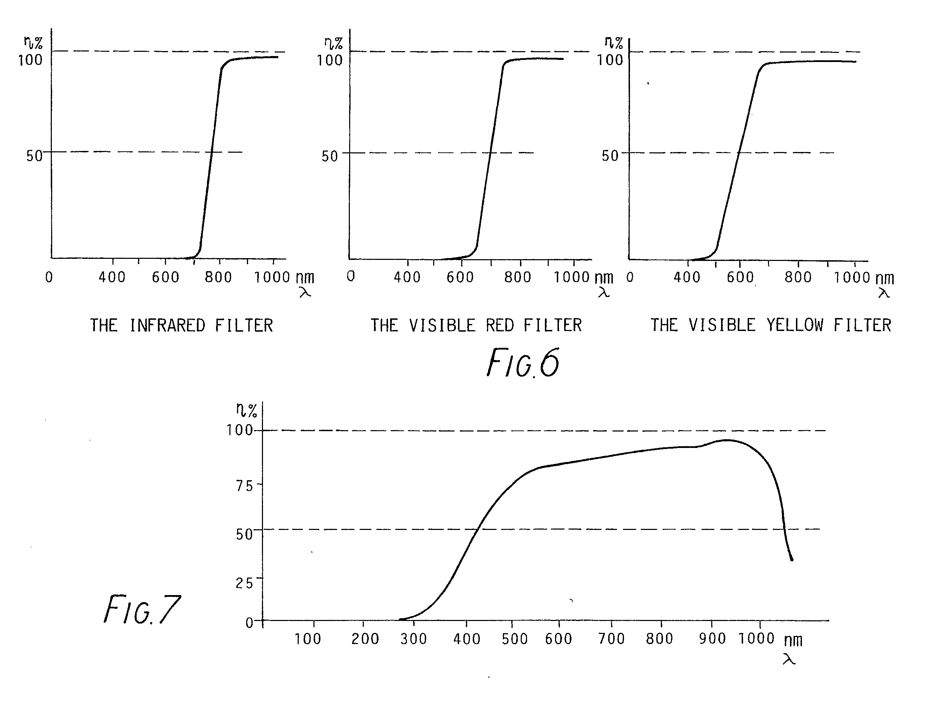

Fig. 5 is the schematic drawing of the compression plate 17. It is made of transparent organic glass with a thickness of 2mm. It is scaled as shown in the central part 18.

Fig. 6 are the curves of transmitting wavelengths respective to each type of cut-off filters. The cut-off wavelength of the infrared filter is 700 nanometers; the cut-off wavelength of the visible red light filter is 650 nanometers; and the cut-off wavelength of the visible yellow light filter is 550 nanometers. All three are optical filters of the cut-off type. As shown on the curves, the absissa axis refers to the wavelengths, while the longitudinal axis presents the transmission ratio.

Fig. 7 is the spectral response region of the CCD detector array. The absissa axis refers to the wavelengths and the longitudinal axis presents the spectral response ratio.

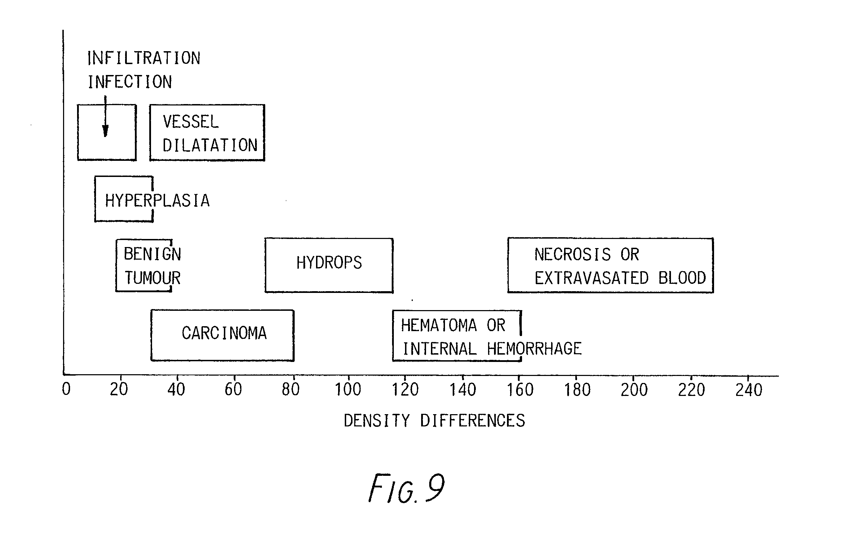

Fig. 8 is the schematic drawing which shows the placing method and the relative positions of the light source and the CCD detector. The diffuser 1 changes the light beam generated by the source with wide bandwidth into diffused light. 20 is the normal direction of the diffuser surface. The compression plate 17 is placed perpendicularly to the diffuser surface. The CCD detector 22 receives images of human tissue 19 through the selected filters 23 in the direction shown in 21. The angles between the direction 21 and the direction 20 should be in the range of 50 to 110 degrees, preferably 90 degrees.

Fig. 9 is the distribution chart of the density differences of the breast lesions, which are calculated by the computer system in comparisons between diseased areas separately in conjunction with their normal areas. Different kinds of lesions have different density difference distributions. The overlapping distributions of different types of lesions can be identified by the image characteristics of each. The density differences beyond the dotted lines are not relevant to the invention.

In Fig. 9 density differences corresponding to various pathological conditions in the breast are indicated, based upon the analysis and classification from medical case histories. The pathological region with regular edges distributing an average density difference from 10 to 35 can be diagnosed as breast proliferation. For example: a patient had a round focus of infection in her left breast; the density difference of it showed as 10, but she had no other symptoms. The focus of infection was diagnosed as proliferation, which was thus identified by pathology after biopsy. The density difference of normal benign fibre gland tumour is from 20 to 40, while that of benign lipoma is a little bit bigger, from 35 to 47, and has other characteristics, such as regular edge and concomitant blood vessel shadow. For example: The characteristics of the lesion of a patient met the density difference of lipoma indicated as 45. The lesion was diagnosed as benign lipoma, and was proved by pathology after operation. The density difference of breast cancer is generally from 33 to 85, and generally concentrates on the difference from 50 to 60. The lesion of a patient during menses was found with the density difference of 92. This was a special case; since the patient was in the period of menses, the congestion of the breast was taken into account. The density difference was modified and ended as 82. With this data and the movable status of the lesion, it was diagnosed as breast cancer but not as hydrops. This was proved by pathology after operation. In aother example, the lesion was detected with a density difference from 63 to 67, and other symptom of it appeared like cancer. The lesion was diagnosed as cancer, and was proved by pathology after operation.

Since water has a greater ability to absorb near infrared light than blood, the density difference shown of the former is less than that of the latter. Generally the density difference of cystic hydrops is from 72 to 115, concentrating usually around the difference from 90 to 100. The density difference of both hematoma and internal hemorrhage is in the range between 105 and 165, and both of them always appear with surrounding blood vessels attached, but other characteristics of the two types of lesions are different and can be identified. For instance, the density difference of a patient's lesion was 133, and was diagnosed as hematoma. After the treatment of pharmacotherapy in combination with massotherapy, the lesion disappeared. For such cases as necrosis and extravasted blood, the density difference of those is even greater and in the range from 150 to 230, and their edges appear very sharp. These lesions can be considered rare.

The density difference of infiltration infection is rather less, generally between 5 and 15, and the edges of the lesion are not clear. For example, a patient had a lesion with a large area but a smaller density difference from 5 to 9. The lesion was diagnosed as infiltration infection, and the patient recovered after pharmacotherapy.

The density difference of vessel dilation is from 30 to 65 and is similar to that of breast cancer. But there is an obvious characteristic in appearance. A patient had a linear lesion, the density difference of which was detected as 32. The lesion was diagnosed as vessel dilation, which was proved by pathology after operation.

Other breast diseases exhibit their own ranges of density differences and all kinds of accompanying characteristics. We can diagnose all kinds of breast diseases, especially breast tumours, according to their data and characteristics. When the present method is applied to detection and diagnosis, the sensitivity achieves 96%; the specificity reaches to 97%; false positive and false negative respectively become 4% and 3%.

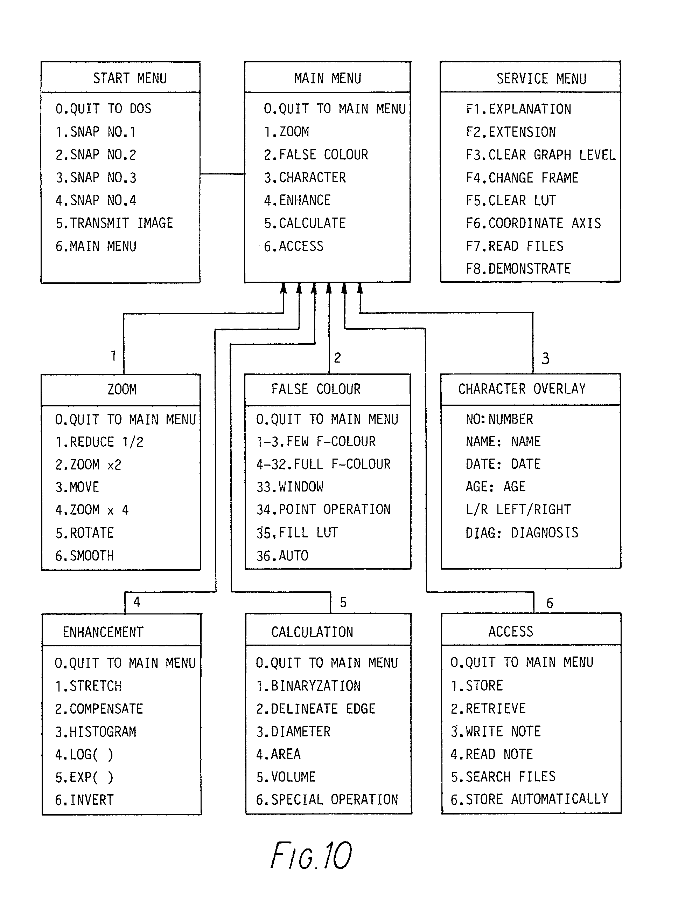

Fig. 10 is the stream diagram of the menu-driven system in support of the system operation. The Start Menu is mainly used to catch images and to copy images, and also used to initialize the system. The Main Menu plays a role to manipulate the sub-menus. The Zoom Sub-menu drives the system functions for geometric processing, such as image enlargement and image reduction. The False Colour Sub-menu drives the functions such as adding false colour onto images, and calculating density value, density difference, etc. The Character Overlay Sub-menu is designed for word processing. The Enhancement Sub-menu drives the functions of various type of image intensity processing, such as increasing the resolution of image, extracting useful information, etc. The Calculation Sub-menu is used for outputting the dimension, the projected area and volume of the diseased area, and locating the lesion area, as well as other image processing. The Service Menu presents all the system functions; the functions used most often can be selected from this menu. Underneath some sub-menus, there are more detailed sub-menus of sub-menus for extending the system functions.

Case 1:

In one general examination of breast disese, an examinee, Ms. Chen, age 46, narrated that she was not aware of any symptom in her breast, and that she felt no pain when pressed. No abnormal symptom was found by hand palpation. Using the present invention, a small shadow was detected on the left side of her left nipple. As the breast was of medium size, light at the sixth level of intensity was applied and the cut-off filter which allows visible red and infrared was selected. By using the compression plate and changing the position of the patient, it was found that the focus of infection was movable and not easily observed. Placing the diffused light at right angle to the CCD detector and adjusting the gain, and slightly pressing the breast with the plate, and by then using image fixation function, a small lesion was detected. The calculated density difference was from 42 to 45. According to other characteristics, the lesion was diagnosed as breast cancer at its early stage, but the right breast was normal. The whole procedure of the examination took less than 4 minutes only.

Case 2:

An associate professor in physical education in Singapore, Ms . Ye, age 48, narrated that she felt dull pain periodically inside both breasts, and that no abnormal phenomena were found in the routine examination at the local hospital. Her breasts are large and dense. Detecting with the present invention, a front position was adopted, a light filter which allows the yellow light to pass through was selected, a light intensity of level 6 was used, and the right side of the breast was pressed with the compression plate. The molar shadow surrounding the blood vessel was then found. When raising the light intensity up to level 7, the molar shadow became clearer. After fixing the image and processing it in the computer, the density difference elicited was from 15 to 19. Analysing the characteristics and ruling out the possibility of infiltration infection, the lesion was diagnosed as a pathological proliferation. It took about 2 minutes to accomplish the diagnosis. The patient was prescribed pharmacotherapy, and after one month, the condition disappeared.

Case 3:

Ms. Wang, age 39, narrated that she had twice felt dull pain. A small, round, soft molar in her breast was detected by palpation. As her breast was of less than medium size, the light intensity at leval 5, a infrared filter, and a side position of the body were selected. When the diffused light was aimed from underneath the right nipple, and the CCD detector was placed right to the right breast, a round shadow with rather sharp edges was observed. After fixing the image at the density difference in the range from 31 to 33, the maximum dimension of 1.4 cm and the projected area of 1.6 cm were outputted. The lesion was diagnosed as a malignant tumor. Afterwards, the diseased area was examined by mammography and diagnosed as pathological proliferation with the possibility of tumor. One month later, the lesion was re-examined with the same approach by the system. The density difference increased up to 42 - 43, the maximum dimension was 1.7 cm, while the projected area became 2.4 cm. Since the lesion had a tendency toward movable status and other charactaristics of cancer, it was diagnosed as breast cancer with all data employed. This was proven by pathology after operation.

Case 4:

Ms. Song, age 34, narrated that there was a small hard lump in the left breast, sometimes detectable by palpation and sometimes not. The breast was medium-dense. Using a filter for the visible red and infrared light, the light intensity at level 5, the compression plate pressing on top of the nipple, and the diffused light illuminating from the left side horizontally, an irregular shadow in the size of a coin was discriminated. When enhancing the intensity of light up to level 6, the irregular shadow became more distinctive. After fixing the image, the density difference of 92 to 95 was provided. The concomitant blood vessel surrounding the dark shadow appeared clearly. The lesion was diagnosed as hematoma. Since the hematoma could be self-absorbed temporarily, it was sometimes undetectable. The whole examination procedure took only 3 minutes . The hematoma was proved by biopsy.

**Claims**

1. A method for human tissue examination and for high-resolution imaging by utilizing non-ionizing radiation characterized in that:

   1) using a diffuser to enable a large area of tissue to be transilluminated with diffused light of a wide spectral bandwidth in visible and near infrared light;

   2) using the said diffused light to transilluminate the tissue being examined, and using a scaled compression plate slightly pressing on the surface of the tissue;

   3) receiving the light passing through the examined object with at least one or more cut-off filters which should allow transmission of light with wavelengths bigger than 550nm, 650nm and 700nm respectively to pass through;

   4) permitting the light transmitting through the cut-off filters to be received by the CCD detector for imaging, while the axis of the CCD detector sits at certain angles to the normal of the diffuser surface.

2. A method for examining human tissue and for high-resolution imaging according to claim 1 characterized in that: using a compression plate to press the examined tissue and to enable the light illuminating the tissue to pass through at an even tissue thickness, while placing the plate perpendicular to the axis of the CCD detector.

3. A method for human tissue examining and for high-resolution imaging according to claim 1 wherein the axis of the CCD detector is placed at certain angles of 50 to 110 degrees, preferably 90 degrees to the normal of the diffuser surface.

4. A method for human tissue examining and for high-resolution imaging according to claims 1, 2, or 3 characterized in that:

   1) inputting images of tissue elicited by the CCD detector into the computer processing system;

   2) processing and analyzing the same images; outputting density differences between normal areas and diseased area as well as the projected areas of lesions; and providing physicians with quantitative information for diagnosing lesions by means of the computer processing system.

5. An apparatus for human tissue examination and for high-resolution imaging by using non-ionizing radiation comprised of a light source, a CCD detector and a computer system, characterized in that:

   1) the light source with a wide spectral band-width emits the visible and the near infrared light;

   2) an optical diffuser is placed between the light source and the examined object,

   3) the selected cut-off filters, which allow the transmitting light with wavelengths respectively over 550nm, 650nm and 700nm to pass through are placed between the examined object and the CCD detector;

   4) the axis of the said CCD detector is placed at certain angles to the normal of the diffuser surface.

6. Apparatus for human tissue examination and for high-resolution imaging according to claim 5, wherein a scaled compression plate is used to slightly press on the examined tissue and to enable the transilluminating light to pass through an even thickness of the tissue, while the plate is placed perpendicular to the axis of the CCD detector.

7. Apparatus according to claim 5 wherein the axis of the said CCD detector is placed at certain angles of about 50 to 110 degrees, preferable 90 degrees, to the normal of the diffuser surface.

8. Apparatus according to claim 5 wherein the said computer processing system contains three image processing board units which are used to handle signals elicited by the CCD detector as well as to calculate the density differences between the normal and the diseased areas in addition to the projected area of the lesion.

9. Apparatus according to claim 5 wherein the said light source is disposed in the light probe, which contains a light bulb, a reflecting cup, an optical diffuser, a light shield, a cooling fan and a sealed heat shield.

10. Apparatus according to claim 9 wherein the said light probe is equipped with an image fixing device which can rapidly and conveniently hold and store desired images as needed by the examiner.

11. Apparatus according to claim 9 wherein the light probe is also equipped with light intensity control facilities which are used to adjust light intensity as needed during examination.

12. Apparatus according to claim 9 wherein the reflecting cup in the shape of parabolic mirror is manufactured with cooling holes, as is likewise the said light shield.

13. Apparatus according to claim 12 wherein the the cooling holes of the said reflecting cup are of oblong shape, and occupy a 15% to 30%, preferably 25%, area of the total reflecting cup; and the central part of the cup with a dimension of 16mm is not manufactured with cooling holes.

14. Apparatus according to claim 5 wherein the operation functions of the said computer system are selected with a menu-driven structure in conjunction with an audio-instruction facility to assist the examiner.

FIG. 1

EP 0 447 708 A2

FIG. 2

FIG. 3

FIG.4

FIG.5

FIG.8

11

THE VISIBLE YELLOW FILTER

THE VISIBLE RED FILTER

THE INFRARED FILTER

*FIG.6*

*FIG.7*

INFILTRATION
INFECTION

VESSEL
DILATATION

HYPERPLASIA

BENIGN
TUMOUR

HYDROPS

CARCINOMA

HEMATOMA OR
INTERNAL HEMORRHAGE

NECROSIS OR
EXTRAVASATED BLOOD

0    20    40    60    80    100    120    140    160    180    200    220    240

DENSITY DIFFERENCES

FIG. 9

| START MENU |
| --- |
| 0.QUIT TO DOS |
| 1.SNAP NO.1 |
| 2.SNAP NO.2 |
| 3.SNAP NO.3 |
| 4.SNAP NO.4 |
| 5.TRANSMIT IMAGE |
| 6.MAIN MENU |

| MAIN MENU |
| --- |
| 0.QUIT TO MAIN MENU |
| 1.ZOOM |
| 2.FALSE COLOUR |
| 3.CHARACTER |
| 4.ENHANCE |
| 5.CALCULATE |
| 6.ACCESS |

| SERVICE MENU |
| --- |
| F1.EXPLANATION |
| F2.EXTENSION |
| F3.CLEAR GRAPH LEVEL |
| F4.CHANGE FRAME |
| F5.CLEAR LUT |
| F6.COORDINATE AXIS |
| F7.READ FILES |
| F8.DEMONSTRATE |

1

| ZOOM |
| --- |
| 0.QUIT TO MAIN MENU |
| 1.REDUCE 1/2 |
| 2.ZOOM x2 |
| 3.MOVE |
| 4.ZOOM x 4 |
| 5.ROTATE |
| 6.SMOOTH |

2

| FALSE COLOUR |
| --- |
| 0.QUIT TO MAIN MENU |
| 1-3.FEW F-COLOUR |
| 4-32.FULL F-COLOUR |
| 33.WINDOW |
| 34.POINT OPERATION |
| 35.FILL LUT |
| 36.AUTO |

3

| CHARACTER OVERLAY |
| --- |
| NO: NUMBER |
| NAME: NAME |
| DATE: DATE |
| AGE: AGE |
| L/R LEFT/RIGHT |
| DIAG: DIAGNOSIS |

4

| ENHANCEMENT |
| --- |
| 0.QUIT TO MAIN MENU |
| 1.STRETCH |
| 2.COMPENSATE |
| 3.HISTOGRAM |
| 4.LOG( ) |
| 5.EXP( ) |
| 6.INVERT |

5

| CALCULATION |
| --- |
| 0.QUIT TO MAIN MENU |
| 1.BINARYZATION |
| 2.DELINEATE EDGE |
| 3.DIAMETER |
| 4.AREA |
| 5.VOLUME |
| 6.SPECIAL OPERATION |

6

| ACCESS |
| --- |
| 0.QUIT TO MAIN MENU |
| 1.STORE |
| 2.RETRIEVE |
| 3.WRITE NOTE |
| 4.READ NOTE |
| 5.SEARCH FILES |
| 6.STORE AUTOMATICALLY |

*FIG.10*

14